# EUROPEAN PATENT APPLICATION

(11) **EP 4 643 860 A1**
(43) Date of publication of application: **05.11.2025**
(21) Application number: 23910781.6
(22) Date of filing: 28.12.2023
(51) Int. Cl.: A61K 31/505, A61P 1/02, A61P 31/04, A61P 35/00, A61P 39/06, A61K 9/00, A61K 8/49, A61Q 11/00

(54) **USE OF ECTOIN OR DERIVATIVE THEREOF IN PREVENTING AND/OR AMELIORATING CELL DAMAGE CAUSED BY IRRITANT, AND METHOD**

(30) Priority: 28.12.2022 CN 202211695288; 20.06.2023 CN 202310735638; 20.06.2023 CN 202310735807
(71) Applicant: Bloomage Biotechnology Corporation Limited, Jinan, Shandong 250101 (CN)
(72) Inventor: DAI, Liyun, Jinan, Shandong 250101 (CN); QU, Wenjie, Jinan, Shandong 250101 (CN); WU, Yue, Jinan, Shandong 250101 (CN); ZOU, Songyan, Jinan, Shandong 250101 (CN); WEN, Liangliang, Jinan, Shandong 250101 (CN)
(74) Representative: Abel & Imray LLP
(86) International application number: PCT/CN2023/142559
(87) International publication number: WO 2024/140864

(57) **Abstract**

A use of ectoin or a derivative thereof in preventing and/or ameliorating cell damage caused by an irritant, the cell damage caused by an irritant comprising oral inflammation, gum damage caused by cigarette smoke, or cell damage caused by acetaldehyde.

## Description

### TECHNICAL FIELD

The present application relates to the technical field of personal care products, and in particular to the use of ectoine or a derivative thereof in preventing and/or ameliorating cell damage caused by an irritant and a method.

### BACKGROUND ART

In daily life, irritants from different sources can cause different cell damages in the human body. For example, oral pathogen lipopolysaccharide (LPS) can induce oral inflammation; UV, irritating chemicals, etc. can cause skin inflammation; cigarette smoke can cause gingival damage; and acetaldehyde can cause oral damage, etc.

There are significant differences in the pathogenic mechanisms and corresponding treatment methods between oral inflammation induced by oral pathogen lipopolysaccharide (LPS) and some skin inflammation. In terms of pathogenicity, since the oral mucosa has histological issues such as lack of a granular layer and a stratum corneum, incomplete cell keratinization, and loose connections between cells compared to the skin, oral microorganisms can easily damage oral tissues and even enter the human body fluid system, inducing many oral problems. In terms of treatment methods, oral inflammation can be effectively alleviated by inhibiting inflammation induced by lipopolysaccharides produced by oral pathogens. However, skin inflammation is not related to the lipopolysaccharides produced by oral pathogens. The above treatment methods for oral inflammation will not necessarily alleviate skin inflammation, and vice versa. Therefore, oral inflammation and skin inflammation are completely different inflammations.

There are also large differences between the LPS produced by different bacterial species that cause inflammation. For example, *Porphyromonas gingivalis* LPS and *Escherichia coli* LPS are different in structure and various biological functions (Bainbridge et al., 2002; Zhang et al., 2008); for another example, *Porphyromonas gingivalis* LPS can induce LBP expression through both TLR2 and TLR4, while *Escherichia coli* LPS can only induce LBP expression through TLR4 (Ding et al., 2012). The existence of these differences makes the inflammatory response of human dental pulp cells caused by the two LPS also very different (Mojtahedi et al., 2022), and ultimately leads to the difference in the host response they induce. Many studies have confirmed that the lipopolysaccharide produced by *Porphyromonas gingivalis* (Pg.LPS) is the main pathogenic component of the bacterial outer membrane. Pg.LPS can directly act on periodontal tissue cells, stimulate effector cells such as monocytes and macrophages, induce inflammatory responses and then cause periodontal tissue damage. Therefore, it is very important to reduce the inflammation induced by Pg.LPS. It can be seen that reducing or inhibiting the inflammation caused by *E. coli* LPS does not necessarily reduce or inhibit the inflammation caused by *Porphyromonas gingivalis* LPS, and vice versa.

Gingival damage caused by cigarette smoke is different from other gingival damage. Two main phases are found in the whole cigarette smoke, one is the tar phase and the other is the gas phase, which is a complex mixture of more than 7,000 compounds. Studies have shown that cigarette smoke extract (CSE) has the ability to degrade collagen and can cause damage to alveolar type II epithelial cells, nuclear factor-κB (NF-κB) activation, and increased secretion of tumor necrosis factor (TNF-α). Smoking reduces fibroblast binding and the production of collagen, as well as the production of bone morphogenetic proteins, and increases the level of tissue destructive enzymes (MMPs). Therefore, gingival damage caused by cigarette smoke is multiple and complex damage.

Gingival damage caused by cigarette smoke is also different from skin damage. In the cavity, the epithelial cells on the mucosal surface are the first line of defense against harmful environmental stimuli such as cigarette smoke. Tissues such as the gingival in the mouth are directly in contact with cigarette smoke, resulting in a series of oral diseases. Epidemiological studies have confirmed that smoking is a recognized risk factor for periodontal disease. In patients with periodontitis, smoking is associated with attachment loss and bone resorption. Smoking can also have a negative impact on the response of periodontal tissues to treatment. Studies have found that cigarette smoke affects gingival blood flow, cytokine production, cell morphology of connective tissue cells (such as fibroblasts), cell migration, proliferation and attachment, and synthesis and secretion of protein.

After daily eating or drinking, the microorganisms in the mouth will utilize alcohol metabolism to produce a large amount of acetaldehyde independently of liver metabolism. At the same time, most alcohol is metabolized by the liver to produce acetaldehyde, which enters the bloodstream and flows to various tissues throughout the body, including the oral cavity. In addition, smoking is also a major source of acetaldehyde in the mouth. Cigarettes produce a large amount of acetaldehyde during the burning process. However, due to the lack of ALDH in the microorganisms in the mouth and the human body, acetaldehyde cannot be further metabolized into harmless acetic acid in time, which leads to a large accumulation of acetaldehyde in the special environment of the mouth, causing serious damage to the mouth.

The damage induced by acetaldehyde is different from conventional wound damage. Common wound damage mainly involves inflammation induced by cell rupture and invasion of external microorganisms. At the same time, the extracellular matrix supporting cells will also be damaged. Therefore, the ingredients conventionally used for wound damage are mainly concentrated in hemostasis, anti-inflammatory, and proliferation. Studies have found that the damage induced by acetaldehyde mainly involves two aspects: oxidative damage and carcinogenesis. Acetaldehyde has obvious cytotoxicity. Acetaldehyde can induce the body to produce a large number of free radicals, causing oxidative damage to macromolecules such as proteins, lipids, and DNA in cells. In addition, acetaldehyde can also covalently bind to DNA to form adducts, which causes cross-linking and breakage of DNA double strands, leading to irreversible cell carcinogenesis. In view of the various hazards of acetaldehyde, how to reduce the damage of acetaldehyde to cell tissues is of great significance.

### SUMMARY

The present application provides the use of ectoine or a derivative thereof in preventing and/or ameliorating cell damage caused by an irritant.

The specific technical solutions of this application are as follows:
1. Use of ectoine or a derivative thereof in preventing and/or ameliorating cell damage caused by an irritant, wherein the cell damage caused by the irritant comprises one or more selected from the group consisting of: oral inflammation, gingival damage caused by cigarette smoke, and cell damage caused by acetaldehyde.
2. The use according to item 1, wherein the oral inflammation is oral inflammation caused by *Porphyromonas gingivalis* lipopolysaccharide.
3. The use according to item 1, wherein the gingival damage caused by cigarette smoke comprises gingival atrophy and/or gingival inflammation.
4. The use according to item 1, wherein the cell damage caused by acetaldehyde comprises oxidative damage caused by acetaldehyde or a cancer caused by acetaldehyde.
5. The use according to item 1 or 2, wherein the oral inflammation is gingivitis caused by *Porphyromonas gingivalis* lipopolysaccharide.
6. The use according to item 1 or 3, wherein the gingival damage comprises gingival fibroblast damage.
7. The use according to item 1 or 4, wherein a site of the cell damage caused by acetaldehyde comprises oral cavity, nasal cavity or gastrointestinal tract.
8. The use according to any one of items 1-7, wherein the ectoine or a derivative thereof is prepared into a product, and the product comprises an oral preparation or an external preparation.
9. A method for preventing and/or ameliorating cell damage caused by an irritant, comprising administrating ectoine or a derivative thereof to an irritated site, wherein the cell damage caused by the irritant comprises oral inflammation, gingival damage caused by cigarette smoke or cell damage caused by acetaldehyde.
10. The method according to item 9, the oral inflammation is oral inflammation caused by Porphyromonas gingivalis lipopolysaccharide.
11. The method according to item 9, wherein the gingival damage caused by cigarette smoke comprises gingival atrophy and/or gingival inflammation.
12. The method according to item 9, the cell damage caused by acetaldehyde comprises oxidative damage caused by acetaldehyde or a cancer caused by acetaldehyde.
13. The method according to item 9 or 10, the oral inflammation is gingivitis caused by *Porphyromonas gingivalis* lipopolysaccharide.
14. The method according to item 9 or 11, the gingival damage comprises gingival fibroblast damage.
15. The method according to item 9 or 12, a site of the cell damage caused by acetaldehyde comprises oral cavity, nasal cavity or gastrointestinal tract.
16. The use according to any one of items 1-8 or the method according to any one of claims 9-15, wherein the derivative of ectoine comprises hydroxyectoine, ectoine sodium salt or ectoine potassium salt.

### Effects of the Invention

The present application discovered that different irritants can cause different cell damages, and ectoine can prevent or ameliorate cell damages caused by a variety of irritants, such as oral inflammation caused by *Porphyromonas gingivalis* lipopolysaccharide, gingival damage caused by cigarette smoke, or cell damage caused by acetaldehyde.

This provides a theoretical basis and foundation for the development of products that prevent or ameliorate cell damage caused by the above-mentioned irritants and for the development and preparation of novel oral care products, and expands the application scope of ectoine or a derivative thereof.

### BRIEF DESCRIPTION OF THE DRAWINGS

The drawings are used to better understand the present application and do not constitute an improper limitation on the present application, wherein
FIG. 1 shows the relative content of ROS in different groups in Example 1.1;
FIG. 2 shows the relative content of TNF-α in different groups in Example 1.2;
FIG. 3 shows the concentration of inflammatory factor IL-6 in each group in Example 2;
FIG. 4 shows the concentration of inflammatory factor IL-6 in each group in Comparative Example 1.

### DETAILED DESCRIPTION

The following provides an explanation of the exemplary embodiments of the present application, including various details to aid understanding, which should be considered as merely exemplary. Therefore, it should be recognized by those of ordinary skill in the art that various changes and modifications can be made to the embodiments described herein without departing from the scope and spirit of the present application. Similarly, for the sake of clarity and conciseness, the description of well-known functions and structures is omitted in the following description.

The present application provides new use of ectoine or a derivative thereof, and a method for achieving the use.

The present application provides use of ectoine or a derivative thereof in preventing and/or ameliorating cell damage caused by an irritant, wherein the cell damage caused by the irritant comprises one or more selected from the group consisting of: oral inflammation, gingival damage caused by cigarette smoke, and cell damage caused by acetaldehyde.

Ectoine, also known as 2-methyl-1,4,5,6-tetrahydropyrimidin-4-carboxylic acid, is an amino acid derivative that exists in microorganisms and belongs to cyclic amino acids. In recent years, many studies have shown that ectoine has the effects of delaying skin aging, preventing ultraviolet rays, anti-oxidation, moisturizing, etc., and is commonly used in skincare products with moisturizing and anti-aging effects.

The derivative of ectoine in the present application is not limited and can be any type of derivatives of ectoine in the art, as long as it can prevent and/or ameliorate cell damage caused by the irritant. The derivative of ectoine includes but not limited to ectoine or ectoine salts modified with chemical groups, etc. In a specific embodiment, the derivative of ectoine is hydroxyectoine, ectoine sodium salt or ectoine potassium salt.

In one aspect, the present application provides the use of ectoine or a derivative thereof in preventing and/or ameliorating gingival damage caused by cigarette smoke.

The gingiva refers to the reddish structure that is closely attached to the periphery of the tooth neck and the adjacent alveolar bone, is composed of stratified squamous epithelium and lamina propria, and is part of the oral mucosa. The gingiva described in this application conforms to the general definition in this field.

Cigarette smoke can cause gingival damage. The application has found through research that ectoine or a derivative thereof can effectively prevent and/or ameliorate gingival damage caused by cigarette smoke, and thus it can be used in products that prevent and/or ameliorate gingival damage caused by cigarette smoke. For example, it can be used in personal care products to alleviate gingival damage caused by cigarette smoke. The personal care products can be oral preparations or external preparations. The specific type of preparations is not limited in the present application, and can be chosen from the existing technology by those skilled in the art according to the needs of use. For example, the oral preparations can be powders, granules, capsules, liquid preparations, suspensions, etc., and the external preparations can be patches, sprays, creams, liquid application preparations, etc.

In some embodiments, the present application provides the use of ectoine or a derivative thereof in the preparation of a product/a personal care product for preventing and/or ameliorating gingival damage caused by cigarette smoke.

The dosage of the ectoine or a derivative thereof in the product/personal care product for preventing and/or ameliorating gingival damage caused by cigarette smoke is not limited in the present application, and can be chosen by those skilled in the art according to the conventional usage dosage of the ectoine or a derivative thereof, for example, by weight percentage, it can be 0.01% to 10%. In some embodiments, the concentration of the ectoine or a derivative thereof is 0.01%, 0.05%, 0.1%, 0.2%, 0.3%, 0.4%, 0.5%, 0.6%, 0.7%, 0.8%, 0.9%, 1.0%, 1.5%, 2.0%, 2.5%, 3.0%, 3.5%, 4.0%, 4.5%, 5.0%, 6.0%, 7.0%, 8.0%, 9.0%, 10.0%, etc.

On the other hand, the present application provides the use of ectoine or a derivative thereof in preventing and/or ameliorating oral inflammation.

In some embodiments, the oral inflammation is oral inflammation caused by *Porphyromonas gingivalis* lipopolysaccharide.

In some embodiments, the oral inflammation is gingivitis caused by *Porphyromonas gingivalis* lipopolysaccharide.

The application has found through research that ectoine or a derivative thereof can effectively prevent and/or ameliorate oral inflammation, and thus it can be used in personal care products for preventing and/or ameliorating oral inflammation. The personal care products can be oral preparations or external preparations.

The present application also provides the use of ectoine or a derivative thereof in the preparation of a personal care product for preventing and/or ameliorating oral inflammation. Further, the present application provides the use of ectoine or a derivative thereof as the sole active ingredient in the preparation of a personal care product for preventing and/or ameliorating oral inflammation. The specific type of preparations is not limited in the present application, and can be chosen from the existing technology by those skilled in the art according to the needs of use. For example, the oral preparations can be powders, granules, capsules, liquid preparations, suspensions, etc., and the external preparations can be patches, sprays, creams, liquid application preparations, etc. In a specific embodiment, the personal care product is an external preparation.

The amount of the ectoine or a derivative thereof in the personal care product for preventing and/or ameliorating oral inflammation is not limited, as long as it can achieve the effect of preventing and/or alleviating oral inflammation. In a specific embodiment, the amount of the ectoine or a derivative thereof in the personal care product is 0.001wt% to 0.1wt%, for example, it can be 0.002wt%, 0.003wt%, 0.004wt%, 0.005wt%, 0.006wt%, 0.007wt%, 0.008wt%, 0.009wt%, 0.01%, 0.015wt%, 0.02wt%, 0.025wt%, 0.03wt%, 0.035wt%, 0.04wt%, 0.045wt%, 0.046wt%, 0.047wt%, 0.048wt%, 0.049wt%, 0.05wt%, 0.06wt%, 0.07wt%, 0.08wt%, 0.09wt%, 0.1wt%. In a preferred embodiment, the amount of the ectoine or a derivative thereof is 0.001wt% to 0.05wt%.

In another aspect, the present application provides the use of ectoine or a derivative thereof in preventing and/or ameliorating cell damage caused by acetaldehyde.

Further, the ectoine or a derivative thereof is used in the preparation of a product for preventing or inhibiting cell damage caused by acetaldehyde.

Further, the ectoine or a derivative thereof is used to prevent or inhibit oxidative damage caused by acetaldehyde or to prevent or inhibit a cancer caused by acetaldehyde.

In some embodiments, a site of the cell damage caused by acetaldehyde comprises oral cavity, nasal cavity or gastrointestinal tract, for example, comprises oral cell damage, nasal cell damage, respiratory tract cell damage, gastric mucosal cell damage, digestive tract cell damage and other cell damage in the body that may be caused by acetaldehyde, preferably oral cell damage.

The inventors of the present application have creatively discovered through a large number of experimental studies that the activity of the cells can be increased by using the ectoine or a derivative thereof to treat cells treated with acetaldehyde, indicating that the ectoine or a derivative thereof can slow down the damage of acetaldehyde to cells and can be used in the preparation of the product for preventing or inhibiting cell damage caused by acetaldehyde.

In some embodiments, the product comprises oral preparations and external preparations. The specific type of preparations is not limited in the present application, and can be chosen from the existing technology by those skilled in the art according to the needs of use. For example, the oral preparations can be powders, granules, capsules, liquid preparations, suspensions, etc., and the external preparations can be patches, sprays, creams, liquid application preparations, etc.

In some embodiments, the product comprises an oral care product.

The present application also provides a product having the function of preventing or inhibiting cell damage caused by acetaldehyde, which contains 0.001%-10% of the ectoine or a derivative thereof.

In some embodiments, the content of the ectoine or a derivative thereof in the product is 0.001%-10%, preferably 0.05%-10%.

For example, the content of the ectoine or a derivative thereof in the product can be 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, 0.1%, 0.5%, 1%, 2%, 3%, 4%, 5%, 6%, 7%, 8%, 9%, 10%, etc.

In the present application, when the product is used directly in cell experiments, the content of the ectoine or a derivative thereof in the product can be 0.001%-0.1%, preferably 0.05%-0.1%. When the product is used as an oral preparation or an external preparation, the content of the ectoine or a derivative thereof may change, but the content of ectoine in the product generally does not exceed 10%.

In some embodiments, the product or personal care product comprises a mouthwash, a toothpaste, a pulvis, a lozenge or an oral film. The preparation method of the mouthwash, toothpaste, pulvis, lozenge or oral film is not limited in the present application, and they can all be prepared by conventional preparation methods in the art.

In some embodiments, the product or personal care product further comprises one or more selected from the group consisting of: an antibacterial agent, an anti-caries agent, an anti-allergic agent, an anti-calculus agent, an anti-inflammatory agent, a whitening agent, and a moisturizing agent.

For example, the ectoine or a derivative thereof can be used in the above-mentioned personal care product together with any one, any two, any three, or any four of the antibacterial agent, anti-caries agent, anti-allergic agent, anti-calculus agent, anti-inflammatory agent, whitening agent, and moisturizing agent, or with all of the above-mentioned ingredients.

In the present application, there is no limitation on the antibacterial agent, which may be a conventional antibacterial agent in the art, for example, the antibacterial agent may be stannous chloride, tetrahydrocurcumin, triclosan, and the like.

In the present application, there is no limitation on the anti-caries agent, which may be an anti-caries agent commonly used in the art, for example, the anti-caries agent may be calcium phosphate, sodium trimetaphosphate, magnesium glycerophosphate, calcium lactophosphate, and the like.

In the present application, there is no limitation on the anti-allergic agent, which may be an anti-allergic agent commonly used in the art, for example, the anti-allergic agent may be dipotassium glycyrrhizinate, potassium fluoride, potassium chloride, and the like.

In the present application, there is no limitation on the anti-calculus agent, which may be an anti-calculus agent commonly used in the art, for example, the anti-calculus agent may be pyrophosphate, tripolyphosphate, citrate, and the like.

In the present application, there is no limitation on the anti-inflammatory agent, which may be an anti-inflammatory agent commonly used in the art, for example, the anti-inflammatory agent may be metronidazole, tinidazole, ornidazole, and the like.

In the present application, there is no limitation on the whitening agent, which may be a whitening agent commonly used in the art, for example, the whitening agent may be peroxide bleaching agent, papain, glucose oxidase, and the like.

In the present application, there is no limitation on the moisturizing agent, which may be a moisturizing agent commonly used in the art, for example, the moisturizing agent may be glycerin, propylene glycol, sorbitol, xylitol, hyaluronic acid, and the like.

In some embodiments, the product or personal care product further comprises one or more selected from the group consisting of: a pH regulator, a thickener, and an osmotic pressure regulator.

The pH regulator may be an acid, an alkali, an inorganic salt, etc. available in the art; the thickener may be hydroxyethyl cellulose, carboxymethyl cellulose and a salt thereof, xanthan gum, etc.; and the osmotic pressure regulator may be an inorganic salt, etc. available in the art.

The present application also provides the use of Ectoine or a derivative thereof for the non-therapeutic purpose of preventing and/or ameliorating gingival damage caused by cigarette smoke.

The preventing and/or ameliorating gingival damage caused by cigarette smoke is for non-therapeutic purpose, that is, the non-therapeutic purpose means it is not for the purpose of the diagnosis or treatment of the disease, and can be for the purpose of prevention, amelioration, relief, alleviation, mitigation, etc.

The present application provides a method for non-therapeutic purpose of preventing and/or ameliorating gingival damage caused by cigarette smoke, which comprises administrating ectoine or a derivative thereof in the oral cavity. The administration method can be any administration method in the art, including but not limited to application, buccal administration, atomization, oral administration, injection, etc.

The administration dosage of ectoine or a derivative thereof in the cavity is not limited in the present application, and can be chosen by those skilled in the art according to the conventional dosage of the ectoine or a derivative thereof, for example, by weight percentage, it can be 0.01% to 10%. In some embodiments, the concentration of ectoine or a derivative thereof is 0.01%, 0.05%, 0.1%, 0.2%, 0.3%, 0.4%, 0.5%, 0.6%, 0.7%, 0.8%, 0.9%, 1.0%, 1.5%, 2.0%, 2.5%, 3.0%, 3.5%, 4.0%, 4.5%, 5.0%, 6.0%, 7.0%, 8.0%, 9.0%, 10.0%, etc.

The gingival damage in the present application comprises but is not limited to one or more of gingival bleeding, gingival oxidation, gingival pain, gingival swelling, gingival atrophy, gingival inflammation, etc. In a specific embodiment, the gingival damage comprises gingival atrophy and/or gingival inflammation.

The gingival damage in the present application comprises but is not limited to one or more of fibroblast damage, lymphocyte damage, plasma cell damage, macrophage damage, etc. in the gingiva. In a specific embodiment, the gingival damage comprises gingival fibroblast damage.

The present application provides a use of ectoine or a derivative thereof in the preparation of a product for preventing or inhibiting gingival damage caused by smoking.

In some embodiments of the present application, the ectoine or a derivative thereof is used as the sole active ingredient for use in the prevention or amelioration of gingival damage caused by smoking/cigarette smoke.

In some embodiments of the present application, the ectoine or a derivative thereof is used as the sole active ingredient for use in the preparation of a product/personal care product for preventing or ameliorating gingival damage caused by smoking/cigarette smoke.

In some embodiments of the present application, the ectoine or a derivative thereof is used as the sole active ingredient for use in the prevention or amelioration of gingival damage caused by cigarette smoke.

In some embodiments of the present application, the ectoine or a derivative thereof is used as the sole active ingredient for use in the non-therapeutic purpose of preventing and/or ameliorating gingival damage caused by cigarette smoke.

In the present application, the ectoine or a derivative thereof is for use in the preparation of the personal care product for preventing and/or ameliorating gingival damage caused by cigarette smoke, or for use in the non-therapeutic purpose of preventing and/or ameliorating gingival damage caused by cigarette smoke, which can significantly inhibit the increase in the content of active oxygen in gingival fibroblasts after oxidative irritation by cigarette smoke extracts. Compared with cells not treated with ectoine or a derivative thereof, the results have extremely significant statistical differences, indicating that ectoine or a derivative thereof can inhibit gingival oxidative damage caused by cigarette smoke extracts and can also inhibit the increase in the content of tumor necrosis factor after irritation by cigarette smoke extracts. Compared with cells not treated with ectoine or a derivative thereof, the results have extremely significant statistical differences. It shows that ectoine or a derivative thereof can inhibit the increase in the content of gingival inflammatory factors caused by cigarette smoke extracts and relieve gingival inflammatory irritation.

The new use of ectoine or a derivative thereof and the method for achieving the use provided in the present application can improve oral health, relieve oral discomfort caused by smoking, etc., and prevent the formation of oral diseases, such as oral tumors, and have broad application prospects.

The present application also provides a use of ectoine or a derivative thereof in preventing and/or alleviating oral inflammation.

Further, the present application provides a use of ectoine or a derivative thereof for the non-therapeutic purpose of preventing and/or alleviating oral inflammation.

The non-therapeutic purpose means it is not for the purpose of the diagnosis or treatment of the disease, but may be for the purpose of prevention, amelioration, relief, alleviation, reduction, etc. Further, the present application provides a use of ectoine or a derivative thereof as the sole active ingredient for the non-therapeutic purpose of preventing and/or alleviating oral inflammation.

Further, the present application provides a method for the non-therapeutic purpose of preventing and/or alleviating oral inflammation, and the method comprises administering the ectoine or a derivative thereof in the oral cavity. The administration method may be any administration method in the art, including but not limited to application, buccal administration, atomization, oral administration, injection, and the like.

The derivative of ectoine in the present application is not limited and can be any type of the derivative of ectoine in the art, as long as it can prevent and/or alleviate oral inflammation, including but not limited to ectoine or ectoine salt modified with chemical groups, etc. In a specific embodiment, the derivative of ectoine is hydroxyectoine, ectoine sodium salt or ectoine potassium salt.

In a specific embodiment, in any use of ectoine or a derivative thereof as described above or the method for achieving the use, the oral inflammation is oral inflammation caused by *Porphyromonas gingivalis* lipopolysaccharide.

In a specific embodiment, in any use of ectoine or a derivative thereof as described above or the method for achieving the use, the oral inflammation is gingivitis caused by *Porphyromonas gingivalis* lipopolysaccharide.

The new use of ectoine or a derivative thereof and the method for achieving the use provided in the present application can improve oral health, relieve oral inflammation, particularly oral inflammation caused by *Porphyromonas gingivalis* lipopolysaccharide, and protect the oral cavity, and have broad application prospects.

The present application further provides a method for preventing or inhibiting cell damage caused by acetaldehyde for non-therapeutic purposes, and the method comprises administrating a product containing ectoine or the above-mentioned product to the site of the damage, which can reduce the cell damage caused by acetaldehyde.

In some embodiments, the product containing ectoine or the above-mentioned product is applied or sprayed to the site of cell damage in the subject, for example, applied or sprayed to the oral cavity of the subject.

The damage induced by acetaldehyde mainly involves two aspects: oxidative damage and carcinogenesis. There is currently no effective treatment for oxidative damage and carcinogenesis. The inventors of the present application creatively discovered that ectoine can slow down the damage caused by acetaldehyde, which provides a new basis for the treatment of oxidative damage and carcinogenesis caused by acetaldehyde.

The present application also provides a method for preventing and/or ameliorating cell damage caused by an irritant as described above, and the method comprises administrating ectoine or a derivative thereof to an irritated site, wherein the cell damage caused by the irritant comprises oral inflammation, gingival damage caused by cigarette smoke, or cell damage caused by acetaldehyde.

### Example

The present application provides a general and/or specific description of the materials and test methods used in the experiments. In the following examples, unless otherwise specified, % means wt%, i.e., weight percentage. The reagents or instruments used without indicating the manufacturer are all conventional reagent products that can be obtained commercially.

### Example 1: Effect of ectoine on cells under CSE Induction

### Experimental Example 1.1: Effect of ectoine on ROS (Reactive Oxygen Species) generation under CSE Induction

Ectoin (purchased from Bloomage Biotechnology) was weighed and dissolved in HGF-1 cell (gingival fibroblast) complete culture medium (containing 10% FBS, 1% penicillin-streptomycin), to prepare 100 µg/mL and 500 µg/mL ectoine solutions, respectively.

Preparation of Cigarette Smoke Extract (CSE): The gas collection bottle was sterilized and the HGF-1 cell culture medium was filled into the gas collection bottle. A certain brand of cigarette was fixed on the long tube end of the gas collection bottle, and the short tube end was connected to a vacuum pump. The vacuum pump was turned on and the cigarette was lighted, so that the cigarette smoke entered the culture medium from the long tube end. In this way, cigarette smoke extract was prepared.

Test method: Preparation of a DCFH-DA solution with a final concentration of 10 µM using phenol red-free culture medium.

HGF-1 cells were seeded into a 96-well plate at a density of 30,000 cells/well and cultured at 37°C under 5% CO₂ for later use. After 24 hours for cell attachment, a blank group, a control group, and an experimental group containing 100µg/mL and 200µg/mL ectoine, respectively, were set up, with 100µL/well. After 24 hours of incubation, 200µL of CSE solution at a concentration of 5% was added to each well (only culture medium was added in the blank group and control group). After 3 hours of incubation, the supernatant was sucked out, and the cells were washed with DPBS solution. 200µL of 10µM DCFH-DA working solution was added. After 30 minutes of incubation, the cells were washed with DPBS solution. The fluorescence intensity was measured at an excitation wavelength of 485nm and an emission wavelength of 538nm. Wherein,
Blank group: no ectoine was added and no CSE treatment was performed.
Control group: No ectoine was added, and CSE treatment was performed.

Experimental group: 100µg/mL and 200µg/mL of ectoine were added, respectively, and CSE treatment was performed.

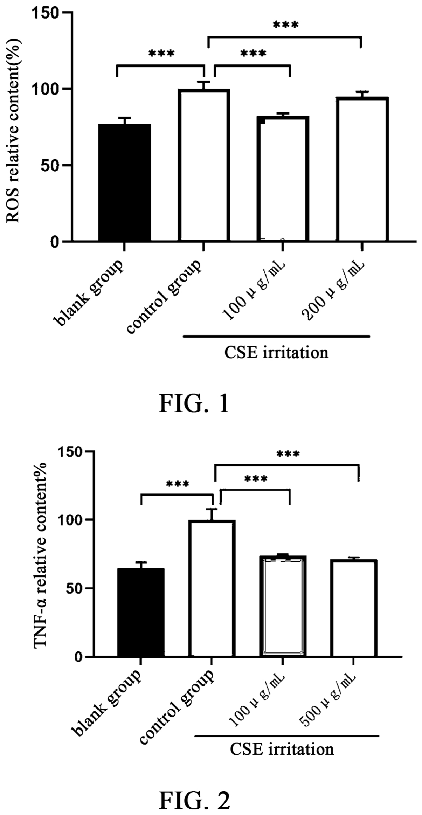

The experimental results are shown in FIG. 1. According to the data analysis of FIG. 1, CSE can significantly stimulate the increase of ROS content in cells, compared to the blank group and the control group. At the same time, it can be seen from the observation of the experimental group and the control group that 100µg/mL and 200µg/mL of ectoine can inhibit the increase of ROS content after CSE oxidative irritation, indicating that it can inhibit the gingival oxidative damage caused by CSE, and the results show statistical difference.

Graphpad Prism statistical software was used for analysis and processing. Pairwise comparison was performed using t-test, wherein *P<0.05 indicates statistical difference, **P<0.01 indicates significant statistical difference, and ***P<0.001 indicates extremely significant statistical difference.

### Experimental Example 1.2: Effect of ectoine on TNF-α (tumor necrosis factor) generation under CSE Induction

Inflammation is closely related to tumors. Tumor necrosis factor α (TNF-α) is one of the main inflammatory cytokines involved in inflammation and is highly expressed in various chronic inflammatory diseases and tumor environments. TNF-α can induce the release of other inflammatory factors to amplify the inflammatory response, and can also directly induce epithelial cell carcinogenesis and promote tumor occurrence by releasing oxygen and nitrogen mediators. This experiment verified the effect of ectoine on gingival inflammation caused by CSE, by the effect of ectoine on TNF-α (tumor necrosis factor) generation under CSE induction.

Ectoine (purchased from Bloomage Biotechnology) was weighed separately and dissolved in HGF-1 cell complete culture medium (containing 10% FBS, 1% penicillin-streptomycin), to prepare 100 µg/mL and 500 µg/mL ectoine solutions, respectively.

Preparation of Cigarette Smoke Extract (CSE): The gas collection bottle was sterilized and the HGF-1 cell culture medium was filled into the gas collection bottle. A certain brand of cigarette was fixed on the long tube end of the gas collection bottle, and the short tube end was connected to a vacuum pump. The vacuum pump was turned on and the cigarette was lighted, so that the cigarette smoke entered the culture medium from the long tube end. In this way, cigarette smoke extract was prepared.

Test method: HGF-1 cells were seeded into a 96-well plate at a density of 30,000 cells/well and cultured at 37°C under 5% CO₂ for later use. After 24 hours for cell attachment, a blank group, a control group, and an experimental group containing 100µg/mL and 500µg/mL ectoine, respectively, were set up, with 100µL/well. After 24 hours of incubation, 200µL of CSE solution at a concentration of 5% was added to each well (only culture medium was added in the blank group and control group). After 24 hours, the supernatant was collected. The secretion of TNF-α was detected using an ELISA detection kit. At the same time as the ELISA detection, cell lysate was added to the 96-well plate and centrifugation was performed, and the supernatant was taken for BCA protein quantification. After homogenization, the control group was used as a benchmark, and the relative content of TNF-α was expressed as a percentage. Wherein,
Blank group: no ectoine was added and no CSE treatment was performed.
Control group: No ectoine was added, and CSE treatment was performed.

Experimental group: 100µg/mL and 500 µg/mL of ectoine were added, respectively, and CSE treatment was performed.

The experimental results are shown in FIG. 2. According to the data analysis of FIG. 2, CSE can significantly stimulate the increase of TNF-α content in cells, compared to the blank group and the control group. At the same time, it can be seen from the observation of the experimental group and the control group that 100µg/mL and 500µg/mL of ectoine can inhibit the increase of TNF-α content after CSE irritation, indicating that it can inhibit the gingival inflammation irritation caused by CSE, and the results show statistical difference.

Graphpad Prism statistical software was used for analysis and processing. Pairwise comparison was performed using t-test, wherein *P<0.05 indicates statistical difference, **P<0.01 indicates significant statistical difference, and ***P<0.001 indicates extremely significant statistical difference.

### Example 2: Effect of ectoine on inflammatory factors induced by Pg.LPS

### Example 2.1

Experimental materials: *Porphyromonas gingivalis* lipopolysaccharide (Pg.LPS) (purchased from Sigma), ectoine (from Bloomage Biotechnology Corporation Limited), and DMEM culture medium.

Experimental sample solution: ectoine and *Porphyromonas gingivalis* lipopolysaccharide were diluted with DMEM culture medium to prepare an experimental sample solution containing a final concentration of 0.001 wt% ectoine + 1 µg/ml Pg.LPS.

CA9-22 cells were cultured in a 24-well plate. After complete attachment, the above experimental sample was added. After 24 hours, the cell culture supernatant was collected. The absorbance at 430 nm and 570 nm was detected by enzyme-linked immunosorbent assay (ELISA), and the concentration of inflammatory factor IL-6 was calculated based on the absorbance.

### Example 2.2

According to the method in Example 2.1, an experimental sample solution containing a final concentration of 0.01wt% ectoine + 1µg/ml Pg.LPS was prepared, and the concentration of the inflammatory factor IL-6 was calculated. Other experimental processes not mentioned were the same as those in Example 2.1.

### Example 2.3

According to the method in Example 2.1, an experimental sample solution containing a final concentration of 0.05wt% ectoine+1µg/ml Pg.LPS was prepared, and the concentration of the inflammatory factor IL-6 was calculated. Other experimental processes not mentioned were the same as those in Example 2.1.

### Comparative Example 2.1

According to the method in Example 2.1, an experimental sample solution containing a final concentration of 1 µg/ml Pg.LPS was prepared, and the concentration of the inflammatory factor IL-6 was calculated. Other experimental processes not mentioned were the same as those in Example 2.1.

### Blank control group

A blank DMEM medium was used as the sample solution, which did not contain ectoine and *Porphyromonas gingivalis* lipopolysaccharide, and the concentration of the inflammatory factor IL-6 was calculated. Other experimental processes not mentioned were the same as those in Example 2.1.

**Table 1 Concentration of the sample solution in each group**

| Group | Concentration of the sample solution |
|---|---|
| Blank control group | / |
| Comparative Example 2.1 | 1µg/ml Pg.LPS |
| Example 2.1 | 0.001wt% ectoine + 1 µg/ml Pg.LPS |
| Example 2.2 | 0.01wt% ectoine + 1µg/ml Pg.LPS |
| Example 2.3 | 0.05wt% ectoine + 1µg/ml Pg.LPS |

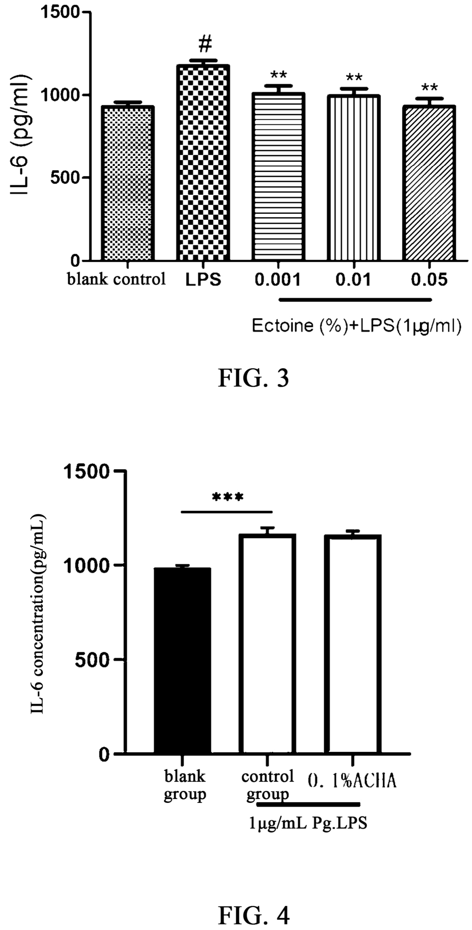

The specific results are shown in FIG. 3. It can be seen from the FIG. 3 that the treatment with 1 µg/ml Pg.LPS alone in Comparative Example 2.1 can cause a significant increase in the IL-6 concentration in CA9-22 cells. Compared with the group treated with Pg.LPS alone, the addition of 0.001wt%, 0.01wt%, and 0.05wt% of ectoine in Examples 2.1, 2.2, and 2.3, respectively, can significantly reduce the concentration of IL-6 caused by Pg.LPS. The results indicate that ectoine can alleviate the inflammation caused by *Porphyromonas gingivalis* lipopolysaccharide.

Statistical analysis: t-test statistical analysis was used to compare each group with group treated with Pg.LPS alone. * indicates p<0.05, which is considered to be of statistical difference. ** indicates p<0.01, which is considered to be of significant statistical difference, and *** indicates p<0.001, which is considered to be of extremely significant statistical difference.

### Example 3: Effect of ectoine on cells treated with acetaldehyde

### Example 3.1

### Experimental materials: ectoine, DMEM culture medium.

Experimental sample solutions: ectoine powder was dissolved in DMEM culture medium to prepare experimental sample solutions with different final concentrations, and the concentrations were shown in Table 2.

Methods: CA9-22 cells purchased from ATCC were co-cultured with different experimental sample solutions for 1 day, then the experimental sample solution was removed and MTT was added. After 3 hours, the supernatant was carefully sucked out and dimethyl sulfoxide (DMSO) was added. The absorbance of each well at 550 nm was detected by a microplate reader. The untreated well was used as the control, the absorbance of the sample well was defined as T, and the absorbance of the untreated well was defined as NT. NT was taken as 100%, and the ratio of T/NT of the sample wells was taken as the cell activity. The cell activity results are shown in Table 2.

**Table 2 Cell activity results**

| Sample | Cell activity |
|---|---|
| Control group | 100% |
| ectoine -0.005wt% | 112% |
| ectoine -0.01wt% | 99% |
| ectoine -0.02wt% | 102% |
| ectoine -0.05wt% | 101% |
| ectoine -0.1wt% | 99% |

It can be seen from the above table that ectoine has no cytotoxicity under the test conditions of this experiment.

### Example 3.2

### Experimental materials: acetaldehyde, DMEM culture medium.

Experimental sample solution: 17.7 M acetaldehyde was diluted with DMEM culture medium to prepare experimental sample solutions with different final concentrations, and the concentrations are shown in Table 2.

Methods: After CA9-22 cells were co-cultured with different experimental sample solutions for 1 day, the experimental sample solutions were removed and MTT was added. After 3 hours, the supernatant was carefully sucked out and dimethyl sulfoxide (DMSO) was added. The absorbance of each well at 550nm was detected by a microplate reader. The untreated well was used as the control, the absorbance of the sample well was defined as T, and the absorbance of the untreated well was defined as NT. NT was taken as 100%, and the ratio of T/NT of the sample well was taken as the cell activity, the results of which are shown in Table 3. On the basis of meeting the requirements of significant difference, the greater the cell activity %, the better the effect of inhibiting cell damage caused by acetaldehyde.

**Table 3 Cell activity results**

| Sample | Cell activity |
|---|---|
| Control group | 100% |
| Acetaldehyde-0.02M | 70% |
| Acetaldehyde-0.01M | 89% |
| Acetaldehyde-0.005M | 93% |
| Acetaldehyde-0.001M | 93% |
| Acetaldehyde-0.0001M | 96% |

It can be seen from the above table that treating cells with different concentrations of acetaldehyde cause cell damage. As the acetaldehyde concentration increases, the damage becomes more obvious. In order to verify the alleviating effect of ectoine on oral damage caused by acetaldehyde, 0.02M of acetaldehyde was selected as the cell damage dose in this application.

### Example 3.3

### Experimental materials: acetaldehyde, ectoine, DMEM culture medium.

Experimental sample solution: ectoine powder and 17.7 M acetaldehyde were diluted with DMEM culture medium respectively, and then the two were mixed in different ratios to prepare experimental sample solutions with different final concentrations. The concentrations are shown in Table 3.

Methods: After CA9-22 cells were co-cultured with different experimental sample solutions for 1 day, the experimental sample solutions were removed and MTT was added. After 3 hours, the supernatant was carefully sucked out and dimethyl sulfoxide (DMSO) was added. The absorbance of each well at 550nm was detected by a microplate reader. The untreated well were used as the control, and the absorbance of the sample well was defined as T, and the absorbance of the untreated well was defined as NT. NT was taken as 100%, and the ratio of T/NT of the sample well was taken as the cell activity. The larger the cell activity %, the better the effect of inhibiting cell damage caused by acetaldehyde.

**Table 4 Cell activity results**

| Sample | Acetaldehyde concentration (M) | Ectoine concentration (wt%) | Cell activity |
|---|---|---|---|
| 1 | / | / | 100% |
| 2 | 0.02 | / | 70% |
| 3 | 0.02 | 0.02 | 72% |
| 4 | 0.02 | 0.03 | 81% |
| 4 | 0.02 | 0.05 | 87% |
| 5 | 0.02 | 0.1 | 88% |

It can be seen from the above table that the cell survival rate of the acetaldehyde-treated group was 70%. After the addition of ectoine, the cell activity of CA9-22 increased. The results indicate that ectoine can reduce the cell damage caused by acetaldehyde.

Comparative Example 1: Effect of acetylated hyaluronic acid (ACHA) on inflammatory factors induced by Pg.LPS

### Experimental group:

Experimental materials: *Porphyromonas gingivalis* lipopolysaccharide (Pg.LPS) (purchased from Sigma), ACHA (from Bloomage Biotechnology Corporation Limited), and DMEM culture medium.

Experimental sample solution: ACHA and Porphyromonas gingivalis lipopolysaccharide were diluted with DMEM culture medium to prepare an experimental sample solution containing a final concentration of 0.1 wt% ACHA + 1 µg/ml Pg.LPS.

CA9-22 cells were cultured in a 24-well plate. After complete attachment, the above experimental sample was added. After 24 hours, the cell culture supernatant was collected. The absorbance at 430 nm and 570 nm was detected by enzyme-linked immunosorbent assay (ELISA), and the concentration of inflammatory factor IL-6 was calculated based on the absorbance.

### Control group:

An experimental sample solution containing a final concentration of 1 µg/ml Pg.LPS was prepared according to the method in the experimental group, and the concentration of the inflammatory factor IL-6 was calculated. Other experimental processes not mentioned were the same as those of the experimental group.

### Blank group:

Blank DMEM medium was used as the sample solution, which did not contain ACHA and Porphyromonas gingivalis lipopolysaccharide, and the concentration of the inflammatory factor IL-6 was calculated. Other experimental processes not mentioned were the same as those of the experimental group.

### Statistical Analysis

Graphpad Prism statistical software was used for analysis and processing. Pairwise comparison was performed using t-test, wherein *P<0.05 indicates statistical difference, **P<0.01 indicates significant statistical difference, and ***P<0.001 indicates extremely significant statistical difference.

**Table 5 Concentration of the sample solution in each group**

| Group | Concentration of the sample solution |
|---|---|
| Blank control group | / |
| Control group | 1µg/ml Pg.LPS |
| Experimental group | 0.1wt% ACHA+ 1µg/ml Pg.LPS |

The specific results are shown in FIG. 4. It can be seen from the FIG. 4 that compared with the blank group, treatment with 1 µg/ml Pg.LPS alone in the control group can cause a significant increase in the IL-6 concentration in CA9-22 cells. Compared with the control group, the addition of 0.1wt% ACHA, which has a certain anti-inflammatory effect, in the experimental group does not significantly reduce the concentration of IL-6 caused by Pg.LPS. The results indicate that although ACHA has an anti-inflammatory effect, it cannot alleviate the inflammation caused by *Porphyromonas gingivalis* lipopolysaccharide.

Although the embodiments of the present application are described above, the present application is not limited to the above specific embodiments and application fields, and the above specific embodiments are merely illustrative and instructive, rather than restrictive. A person of ordinary skill in the art can make many forms under the inspiration of this specification and without departing from the scope of protection of the claims of the present application, all of which fall into the protection of the present application.

## Claims

1. Use of ectoine or a derivative thereof in preventing and/or ameliorating cell damage caused by an irritant, wherein the cell damage caused by the irritant comprises one or more selected from the group consisting of: oral inflammation, gingival damage caused by cigarette smoke, and cell damage caused by acetaldehyde.

2. The use according to claim 1, wherein the oral inflammation is oral inflammation caused by *Porphyromonas gingivalis* lipopolysaccharide.

3. The use according to claim 1, wherein the gingival damage caused by cigarette smoke comprises gingival atrophy and/or gingival inflammation.

4. The use according to claim 1, wherein the cell damage caused by acetaldehyde comprises oxidative damage caused by acetaldehyde or a cancer caused by acetaldehyde.

5. The use according to claim 1 or 2, wherein the oral inflammation is gingivitis caused by *Porphyromonas gingivalis* lipopolysaccharide.

6. The use according to claim 1 or 3, wherein the gingival damage comprises gingival fibroblast damage.

7. The use according to claim 1 or 4, wherein a site of the cell damage caused by acetaldehyde comprises oral cavity, nasal cavity or gastrointestinal tract.

8. The use according to any one of claims 1-7, wherein the ectoine or a derivative thereof is prepared into a product, and the product comprises an oral preparation or an external preparation.

9. A method for preventing and/or ameliorating cell damage caused by an irritant, comprising administrating ectoine or a derivative thereof to an irritated site, wherein the cell damage caused by the irritant comprises oral inflammation, gingival damage caused by cigarette smoke or cell damage caused by acetaldehyde.

10. The method according to claim 9, the oral inflammation is oral inflammation caused by *Porphyromonas gingivalis* lipopolysaccharide.

11. The method according to claim 9, wherein the gingival damage caused by cigarette smoke comprises gingival atrophy and/or gingival inflammation.

12. The method according to claim 9, wherein the cell damage caused by acetaldehyde comprises oxidative damage caused by acetaldehyde or a cancer caused by acetaldehyde.

13. The method according to claim 9 or 10, wherein the oral inflammation is gingivitis caused by *Porphyromonas gingivalis* lipopolysaccharide.

14. The method according to claim 9 or 11, wherein the gingival damage comprises gingival fibroblast damage.

15. The method according to claim 9 or 12, a site of the cell damage caused by acetaldehyde comprises oral cavity, nasal cavity or gastrointestinal tract.

16. The use according to any one of claims 1-8 or the method according to any one of claims 9-15, wherein the derivative of ectoine comprises hydroxyectoine, ectoine sodium salt or ectoine potassium salt.
